(19) Europäisches Patentamt
European Patent Office
Office européen des brevets

(11) Veröffentlichungsnummer: **0 321 857 B1**

(12) **EUROPÄISCHE PATENTSCHRIFT**

(45) Veröffentlichungstag der Patentschrift: **30.03.94**

(51) Int. Cl.[5]: **C07C 51/09**, C07C 65/105, C07C 65/03, C07C 65/05, C07C 65/24, C07C 65/11

(21) Anmeldenummer: **88120961.3**

(22) Anmeldetag: **15.12.88**

Die Akte enthält technische Angaben, die nach dem Eingang der Anmeldung eingereicht wurden und die nicht in dieser Patentschrift enthalten sind.

(54) **Verfahren zur Herstellung von aromatischen Hydroxycarbonsäuren.**

(30) Priorität: **22.12.87 DE 3743517**

(43) Veröffentlichungstag der Anmeldung:
**28.06.89 Patentblatt 89/26**

(45) Bekanntmachung des Hinweises auf die Patenterteilung:
**30.03.94 Patentblatt 94/13**

(84) Benannte Vertragsstaaten:
**CH DE FR GB IT LI**

(56) Entgegenhaltungen:
**EP-A- 0 170 483**
**EP-A- 0 213 340**

(73) Patentinhaber: **BASF Aktiengesellschaft**
**Carl-Bosch-Strasse 38**
**D-67063 Ludwigshafen(DE)**

(72) Erfinder: **Neumann, Peter, Dr.**
**Poststrasse 28**
**D-6800 Mannheim 31(DE)**
Erfinder: **Eichenauer, Ulrich, Dr.**
**Paul-Ehrlich-Strasse 25a**
**D-6000 Frankturt 70(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein neues Verfahren zur Herstellung von aromatischen Hydroxycarbonsäuren durch Oxidation der entsprechenden Ketone, bei denen die Hydroxygruppen acyliert sind, mittels Sauerstoff in Gegenwart eines Katalysators und eines Lösungsmittels, zu aromatischen Hydroxycarbonsäuren, bei denen die Hydroxygruppen acyliert sind, und anschließender Abspaltung der Acylgruppen.

Es ist bekannt, einfache aromatische Hydroxycarbonsäuren durch die Kolbe-Reaktion, d. h. Carboxylierung der entsprechenden Phenolate mit Kohlendioxid, herzustellen.

Es ist weiterhin bekannt, daß aromatische Carbonsäuren aus Acetophenonen mittels der Haloform-Reaktion, d. h. Umsetzung mit Halogenen im alkalischen Medium, erhalten werden. Dieses häufig mit Natriumhypochlorit durchgeführte Verfahren liefert bei solchen Aromaten, die durch elektronenliefernde Gruppen substituiert sind, eine Reihe von Nebenreaktionen. Beispielsweise können alkalisch hydrolysierbare Schutzgruppen abgespalten werden oder es kann Kernhalogenierung oder -oxidation eintreten.

Die EP-A-170 483 beschreibt u.a. die Herstellung von 4-Acetyloxybenzoesäure durch Oxidation von 4-Acetyloxyacetophenon mittels Sauerstoff in Gegenwart eines Mangansalzes als Katalysator und Essigsäure als Lösungsmittel.

Auch aus der EP-A-213 340 ist die Oxidation von verschiedenen Acetophenonen mittels Sauerstoff in Gegenwart von Mangansalzen als Katalysator und Essigsäure als Lösungsmittel bekannt.

Aufgabe der vorliegenden Erfindung war es, ein neues Verfahren bereitzustellen, das in vorteilhafter Weise die Herstellung von aromatischen Hydroxycarbonsäuren erlaubt, wobei die Bildung von Nebenprodukten möglichst ausgeschlossen sein sollte.

Es wurde nun gefunden, daß die Herstellung von aromatischen Hydroxycarbonsäuren der Formel I

$(HO-)_nA-COOH$ (I),

in der n 1 oder 2 und A einen Rest aus der Benzol-, Naphthalin-, Diphenyl-, Diphenylether-, Diphenylsulfid- oder Diphenylsulfon-Reihe bedeuten, wobei man eine acylierte aromatische Verbindung der Formel II

$(R^1-CO-O-)_nA-CO-R^2$ (II),

in der $R^1$ und $R^2$ gleich oder verschieden sind und unabhängig voneinander jeweils gegebenenfalls substituiertes $C_1$-$C_4$-Alkyl bedeuten und n und A jeweils die obengenannte Bedeutung besitzen, mit Sauerstoff in Gegenwart eines Salzes von Vanadium, Mangan, Eisen oder Kobalt als Katalysator und eines Lösungsmittels bei einer Temperatur von 20 bis 250 °C zu einer Carbonsäure der Formel III

$(R^1-CO-O-)_nA-COOH$ (III),

in der $R^1$, n und A jeweils die obengenannte Bedeutung besitzen, oxidiert und anschließend die Acylgruppe(n) abspaltet, vorteilhaft gelingt, wenn man Propionsäure als Lösungsmittel verwendet.

Aus der EP-A-216 279 ist bereits ein Verfahren bekannt, bei dem 5-Chlorthiophen-2-carbonsäure und 3-Chlorthiophen-2-carbonsäure durch Oxidation der entsprechenden 2-Acetylverbindungen mittels Sauerstoff in Gegenwart von Manganacetat oder Kobaltacetat als Katalysator in Eisessig hergestellt werden.

Bei der Oxidation der acylierten aromatischen Verbindungen II unter den genannten Bedingungen war nun zu erwarten, daß ebenso wie die Acylgruppe $-CO-R^2$ zur Carboxylgruppe oxidiert wird, auch die Acyloxygruppe $-O-CO-R^1$ oxidert würde, wobei Produkte mit freien phenolischen OH-Gruppen aufträten, die bekanntlich der oxidativen weiterreaktion zugänglich sind, wobei üblicherweise der aromatische Ring oxidiert wird (siehe Houben-Weyl "Methoden der organischen Chemie", Band 7/3 b, Seiten 15 bis 17).

Es war daher überraschend, daß unter den Bedingungen des erfindungsgemäßen Verfahrens die Acyloxygruppe $-O-CO-R^1$ nicht oxidativ abgebaut wird. Weitherhin war es nicht vorhersehbar, daß keine Nebenprodukte gebildet werden, bei denen, unabhängig von der Acyloxygruppe, eine Oxidation des aromatischen Kerns erfolgt.

Der aromatische Rest A in der Formel I leitet sich von jeweils gegebenenfalls substituiertem Benzol, Naphthalin, Diphenyl, Diphenylether, Diphenylsulfid oder Diphenylsulfon ab.

Als Substituenten kommen dabei beispielsweise Halogen, z. B. Fluor, Chlor oder Brom, Nitro, Carboxy oder Hydroxysulfonyl in Betracht.

Wenn n 1 ist, kommen als zweiwertige Reste -A- beispielsweise

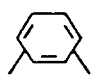 , 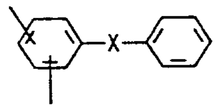 , 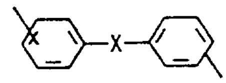 ,

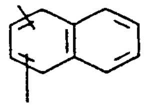 oder 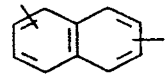 ,

in Betracht, wobei X für eine chemische Bindung O, S oder $SO_2$ steht, und, wie oben ausgeführt, die aromatischen Kerne noch substituiert sein können.

Entsprechende dreiwertige Molekülfragmente liegen vor, wenn n 2 beträgt, wobei auch hier das aromatische System noch substituiert sein kann und bei mehrkernigen Systemen die acylierten Hydroxygruppen auch an verschiedenen aromatische Kerne gebunden sein können.

$R^1$ und $R^2$ stehen beispielsweise für Methyl, Ethyl, Propyl, Isopropyl, Butyl, Isobutyl oder tert-Butyl.

Wenn die Reste $R^1$ und/oder $R^2$ substituiert sind, kommen als Substituenten z. B. Halogen, insbesondere Chlor, $C_1$-$C_4$-Alkoxy, wie Methoxy, Ethoxy, Propoxy, oder Butoxy, oder $C_1$-$C_4$-Alkanoyloxy, wie Formyloxy, Acetyloxy, Propionyloxy oder Butyryloxy in Betracht.

Solchermaßen substituierte Reste $R^1$ und $R^2$ sind insbesondere Chlormethyl, Methoxymethyl, Ethoxymethyl, Formyloxymethyl, Acetyloxymethyl oder Propionyloxymethyl.

Bevorzugt werden solche acylierte aromatische Verbindungen der Formel II oxidiert, in der $R^1$ und $R^2$ unabhängig voneinander Methyl oder Ethyl bedeutet.

Weiterhin bevorzugt werden solche acylierte aromatische Verbindungen der Formel II oxidiert, in der A

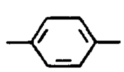 oder 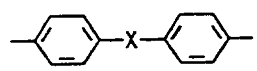

bedeutet, wobei X für eine chemische Bindung oder Sauerstoff steht.

Als Lösungsmittel dient Propionsäure.

Als Katalysatoren dienen die Salze von Vanadium, Mangan, Eisen oder Kobalt. Geeignete Anionen sind z. B. Phosphat, Sulfat, Halogenid, wie Fluorid, Chlorid oder Bromid, oder Carboxylate, wie Formiat, Acetat, Propionat oder Butyrat. Vorzugsweise verwendet man die jeweiligen Sulfate, Halogenide oder Acetate. Ganz besonders hervorzuheben sind diejenigen der genannten Metallacetate, in denen die Oxidationsstufe des jeweiligen Metalls + 2 beträgt.

In einer bevorzugten Ausführungsform verwendet man als Katalysatoren Mischungen der genannten Metallsalze, wobei insbesondere Kobalt- und Mangansalze als Mischungspartner zu nennen sind.

Die Menge der zu verwendenden Katalysatoren ist in einem weiten Bereich variierbar. Besonders zweckmäßig verwendet man die Katalysatoren in einer Menge von $5 \cdot 10^{-3}$ bis $5 \cdot 10^{-1}$ Mol Metallsalz, bezogen auf 1 Mol Verbindung II.

Eine bevorzugte Verfahrensweise besteht darin, die Oxidation in Gegenwart eines Carbonsäureanhydrids, das 3 bis 8 Kohlenstoffatome aufweist, durchzuführen.

In der Regel wird das Carbonsäureanhydrid dabei in einer Menge von 0,5 bis 3 Mol, vorzugsweise 1 bis 2 Mol, jeweils bezogen auf 1 Mol der acylierten aromatischen Verbindung II, zugegeben.

Geeignete Carbonsäureanhydride sind z.B. Acetanhydrid, Propionsäureanhydrid oder die gemischten Anhydride aus Ameisensäure und Essigsäure, Ameisensäure und Propionsäure oder Essigsäure und Propionsäure.

Die Reaktionstemperatur beträgt 50 bis 250 °C, wobei eine Temperatur von 90 °C bis zum Siedepunkt des Reaktionsgemischs bevorzugt ist. Man arbeitet dabei in der Regel bei einem Druck von 0,9 bis 200 bar, wobei ein Reaktionsdruck von 1 bar bevorzugt ist.

Als Oxidationsmittel dient erfindungsgemäß Sauerstoff. Dabei kann der Sauerstoff in reiner Form oder in Mischung mit Inertgasen, beispielsweise Stickstoff angewendet werden. Bevorzugt ist die Verwendung von Luft als Oxidationsmittel. Die Begasung erfolgt dabei zweckmäßig über eine Bodenventil-Düse.

Das erfindungsgemäße Verfahren, das sowohl in kontinuierlicher als auch in diskontinuierlicher Arbeitsweise betrieben werden kann, wird zweckmäßig so durchgeführt, daß man in einem geeigneten Reaktor, z.

B. in einem Flanschreaktor, die acylierte aromatische Verbindung II, gegebenenfalls das Carbonsäureanhydrid, Propionsäure und den Katalysator vorlegt und unter Rühren auf die erfindungsgemäße Temperatur erhitzt, wobei gleichzeitig Sauerstoff durch das Reaktionsgemisch geleitet wird. Nach einer Reaktionszeit, die im allgemeinen 2 bis 10 Stunden beträgt, ist die Oxidation beendet und nach dem Abkühlen des Reaktionsgemischs wird dieses filtriert und der Filterrückstand mit Wasser gewaschen und getrocknet. Es ist auch möglich, das Reaktionsgemisch zunächst in Wasser zu geben und danach die Filtration vorzunehmen.

Der so behandelte Filterrückstand besteht im wesentlichen aus der acylierten Hydroxycarbonsäure III, bei der man nun nach an sich bekannten Methoden die Acylgruppe(n) abspaltet. Dazu kann man die Verbindung III zusammen mit Kalium- oder Natriumhydroxid in Wasser erhitzen oder zusammen mit verdünnten Mineralsäuren, z. B. verdünnter Salzsäure oder verdünnter Schwefelsäure in der Wärme behandeln. Die Abspaltung der O-Acylgruppe kann aber auch durch Umesterung, beispielsweise mit Methanol oder Ethanol erfolgen.

Die acylierten aromatischen Verbindungen der Formel II sind nach Literaturverfahren leicht zugänglich. Beispielsweise können Phenole in einer Acylierungsreaktion nach Friedel-Crafts zu entsprechend C-, O-diacylierten Derivaten umgewandelt werden (siehe z. B. die Umsetzung von 4-Hydroxydiphenyl zu 1-Acetoxy-4'-acetyldiphenyl - Tetrahedron Band 32, S. 1835, 1976). Eine weitere Synthesemöglichkeit ist beispielsweise die Fries-Verschiebung (siehe dazu Org. Reactions Vol. 1, S. 342, 1942), d. h. die Umlagerung von acylierten Phenolen zu Hydroxyacetophenonen, mit anschließender O-Acylierung nach Standardmethoden.

Die mittels des erfindungsgemäßen Verfahrens erhaltenen aromatischen Hydroxycarbonsäuren der Formel I sind beispielsweise wertvolle Monomere zur Herstellung von thermotropen flüssigkristallinen Polymeren.

Die folgenden Beispiele sollen die Erfindung näher erläutern.

Beispiel 1

In einem doppelwandigen 1 1-Flanschreaktor mit Intensivrührer und Stromstörer wurden 500 ml Propionsäure, 50,8 g 4-Acetoxy-4'-acetyldiphenyl, 0,8 g Kobalt(II)acetat und 12,0 g Mangan(II)acetat unter Rückfluß erhitzt und über eine Bodenventildüse mit Luft begast. Nach 4 Stunden wurde abgekühlt, abgesaugt, mit Wasser gewaschen und getrocknet. Ausbeute: 37,9 g (74 %) 4-Acetoxybiphenyl-4'-carbonsäure, Reinheit > 98 % (HPLC).

Beispiel 2

25 g 4-Acetoxybiphenyl-4'-carbonsäure wurden in 100 ml 5 gew.-%iger Natronlauge 1 Stunde auf 80 °C erwärmt. Das Reaktionsgemisch wurde heiß mit konz. Salzsäure angesäuert, der Rückstand abfiltriert, mit Wasser gewaschen und getrocknet. Ausbeute: 20,6 g (98 %) 4-Hydroxydiphenyl-4'-carbonsäure, Reinheit > 99 % (HPLC).

Beispiel 3

345 g 4-Acetoxy-4'-acetylbiphenyl wurden in 2,6 l Propionsäure suspendiert. Zu dieser Suspension wurden 2,6 g Kobalt(II)acetat, 10,1 g Mangan(II)acetat und 375 g Acetanhydrid zugegeben und das Gemisch auf 110 °C erwärmt. Nach 15-minütigem Rühren bei 110 °C wurde über eine Bodenventildüse mit Luft begast. Nach 90-minütigem Begasen bei 110 °C war die Reaktion beendet; man ließ das Reaktionsgemisch über das Bodenventil ab, saugte ab und wusch mit Petrolether nach. Ausbeute: 291,3 g (84 %) Acetoxybiphenylcarbonsäure, Reinheit 99,9 % (HPLC).

In analoger Weise werden die in der Tabelle genannten acylierten aromatischen Verbindungen II in die Hydroxycarbonsäuren I übergeführt.

Tabelle

| Bsp.-Nr. | II | I |
|---|---|---|
| 4 | 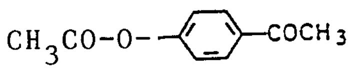 | 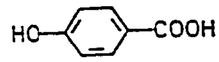 |
| 5 | 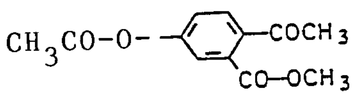 | 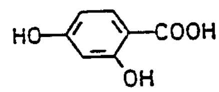 |
| 6 | 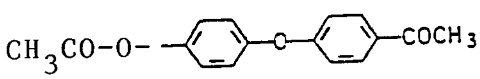 | 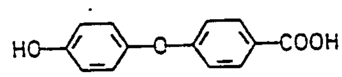 |
| 7 | 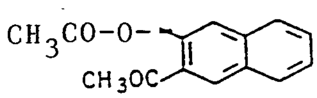 | 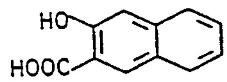 |
| 8 | 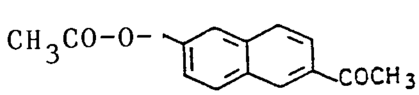 | 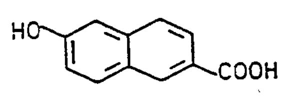 |
| 9 | 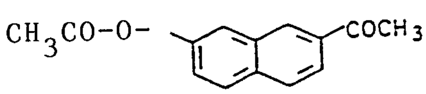 | 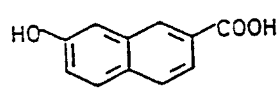 |

## Patentansprüche

**1.** Verfahren zur Herstellung von aromatischen Hydroxycarbonsäuren der Formel I

$(HO-)_nA-COOH$ (I),

in der n 1 oder 2 und A einen Rest aus der Benzol-, Naphthalin-, Diphenyl-, Diphenylether-, Diphenylsulfid- oder Diphenylsulfon-Reihe bedeuten, wobei man eine acylierte aromatische Verbindung der Formel II

$(R^1-CO-O-)_nA-CO-R^2$ (II),

in der $R^1$ und $R^2$ gleich oder verschieden sind und unabhängig voneinander jeweils gegebenenfalls substituiertes $C_1$-$C_4$-Alkyl bedeuten und n und A jeweils die obengenannte Bedeutung besitzen, mit Sauerstoff in Gegenwart eines Salzes von Vanadium, Mangan, Eisen oder Kobalt als Katalysator und eines Lösungsmittels bei einer Temperatur von 20 bis 250 °C zu einer Carbonsäure der Formel III

$(R^1-CO-O-)_nA-COOH$ (III),

in der $R^1$, n und A jeweils die obengenannte Bedeutung besitzen, oxidiert und anschließend die Acylgruppe(n) abspaltet, dadurch gekennzeichnet, daß man Propionsäure als Lösungsmittel verwendet.

**2.** Verfahren gemäß Anspruch 1, dadurch gekennzeichnet, daß man die Oxidation in Gegenwart eines Carbonsäureanhydrids, das 3 bis 8 Kohlenstoffatome aufweist, durchführt.

## Claims

1. A process for the preparation of an aromatic hydroxycarboxylic acid of the formula I

   $(HO-)_nA-COOH$ (I)

   where n is 1 or 2 and A is a radical from the benzene, naphthalene, biphenyl, diphenyl ether, diphenyl sulfide or diphenyl sulfone series, by oxidizing an acylated aromatic compound of the formula II

   $(R^1-CO-O-)_nA-CO-R^2$ (II)

   where $R^1$ and $R^2$ are identical or different and independently of one another are each unsubstituted or substituted $C_1$-$C_4$-alkyl and n and A each have the above-mentioned meanings, with oxygen in the presence of a salt of vanadium, manganese, iron or cobalt as a catalyst and of a solvent at from 20 to 250 °C to give a carboxylic acid of the formula III

   $(R^1-CO-O-)_nA-COOH$ (III)

   where $R^1$, n and A each have the abovementioned meanings, and then eliminating the acyl group(s) wherein the solvent used is propionic acid.

2. The process as claimed in claim 1, wherein the oxidation is carried out in the presence of a carboxylic anhydride of 3 to 8 carbon atoms.

## Revendications

1. Procédé de préparation d'acides hydroxycarboxyliques aromatiques de formule I

   $(HO-)_nA-COOH$ (I),

   dans laquelle n est 1 ou 2 et A est un reste choisi dans la série des benzène, naphtalène, diphényle, oxyde de diphényle, sulfure de diphényle ou sulfone de diphényle, dans lequel on oxyde un composé aromatique acylé de formule II

   $(R^1-CO-O-)_nA-CO-R^2$ (II),

   dans laquelle $R^1$ et $R^2$ sont identiques ou différents et représentent chaque fois indépendamment l'un de l'autre un groupement alkyle en $C_1$-$C_4$ éventuellement substitué, et n et A ont chaque fois la signification susmentionnée, avec de l'oxygène en présence d'un sel de vanadium, manganèse, fer ou cobalt en tant que catalyseur et d'un solvant a une température de 20 à 250 °C, en un acide carboxylique de formule III

   $(R^1-CO-O)_nA-COOH$ (III),

   dans laquelle $R^1$, n et A ont chaque fois la signification susmentionnée, puis on élimine le ou les groupements acyle, caractérisé en ce que l'on utilise comme solvant l'acide propionique.

2. Procédé selon la revendication 1, caractérisé en ce que l'on effectue l'oxydation en présence d'un anhydride d'acide carboxylique, qui présente 3 à 8 atomes de carbone.